# EUROPEAN PATENT SPECIFICATION

(11) **EP 3 039 117 B1**
(45) Date of publication and mention of the grant of the patent: **16.10.2019**
(21) Application number: 14839911.6
(22) Date of filing: 21.08.2014
(51) Int. Cl.: C12M 1/26, C12M 1/24, B01L 3/00, B01D 21/26, B01L 9/00, G01N 1/28, G01N 35/10, C12M 1/30

(54) **APPARATUS FOR SPREADING A FLUID ACROSS A SUBSTRATE**
VORRICHTUNG ZUM VERTEILEN EINER FLÜSSIGKEIT ÜBER EIN SUBSTRAT
APPAREIL POUR ÉTALER UN FLUIDE SUR UN SUBSTRAT

(30) Priority: 26.08.2013 US 201361869866 P
(43) Date of publication of application: 06.07.2016
(73) Proprietor: Rarecyte, Inc., Seattle, WA 98121 (US)
(72) Inventor: NORDBERG, Joshua, Bainbridge Island, Washington 98110 (US); CAMPTON, Daniel, Seattle, WA 98121 (US); QUARRE, Steven, Woodinville, Washington 98072 (US); STEWART, David, Seattle, Washington 98118 (US)
(74) Representative: J A Kemp
(86) International application number: PCT/US2014/052109
(87) International publication number: WO 2015/031158

(56) References cited:
- EP-A1- 2 565 617
- EP-A1- 2 565 617
- EP-A2- 0 806 647
- NL-A- 8 503 194
- US-A- 3 880 111
- US-A- 4 392 450
- US-A- 4 392 450
- US-A- 4 516 522
- US-A1- 2002 132 222
- US-A1- 2013 078 734

## Description

### TECHNICAL FIELD

This disclosure relates to smearing a fluid and, in particular, to spreading a fluid across a substrate.

### BACKGROUND

Analysis of a biological sample includes the step of placing the biological sample on a substrate, such as a microscope slide, for examination. Once placed on the substrate, the biological sample may be smeared across the substrate, thereby forming a wider and thinner layer of the biological sample to enable more efficient processing, imaging and examination of components of the biological sample. However, nonuniformity may lead to problems with imaging and examination, thereby leading to errors and incorrect analysis. As a result, practitioners, researchers, and those working with suspensions or solutions continue to seek an apparatus and method for repeatedly and uniformly spreading a biological sample, a solution, or a suspension across a substrate.

US 2013/078734 A1 discloses a microscope slide cover with integrated reservoir.

NL 8 503 194 A discloses a liquid sample disperser for microscope slides, having a base with slide-locating projections and a linearly-guided slidable spreader with transverse rib on underside.

EP 2 565 617 A1 discloses a smear preparation device comprising a lower unit on which a slide is placed and an upper unit movably connectable to the lower unit. US 4 392 450 A discloses a device for spreading monolayered films.

### SUMMARY OF THE INVENTION

According to the present invention there is provided the system of claim 1. Additional aspects of the invention are set out in the dependent claims.

### DESCRIPTION OF THE DRAWINGS

Figure 1A-1C show an example spreader.
Figures 2A-2B show an example wiper.
Figure 2C shows an example wiper.
Figure 3 shows an example frame.
Figures 4A-4B show an example spreader.
Figure 5 shows an example spreader.
Figure 6 shows an example wiper.
Figure 7 shows a flow diagram for spreading a sample with a spreader.

### DETAILED DESCRIPTION

This disclosure is directed to fluid spreaders and methods for spreading a fluid across a substrate. A spreader includes a wiper and a frame. The wiper and the frame may mate to permit translation of the wiper across, and with respect to, the frame. The wiper spreads the fluid across the substrate and the frame supports the wiper and the substrate. The frame may also include a ramp to cause a portion of the wiper to lift away from the substrate.

### Spreader

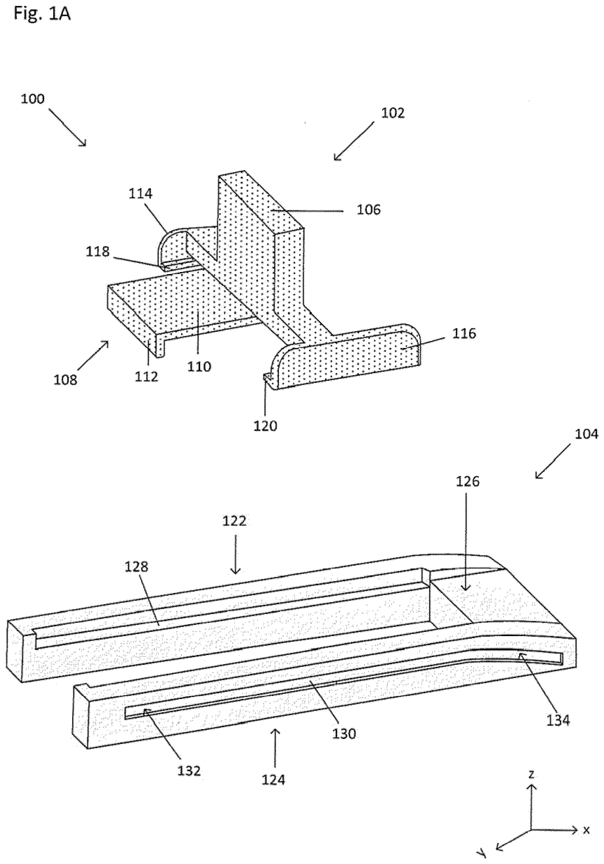

Figure 1A shows an exploded view of a spreader 100. The spreader 100 includes a wiper 102 and a frame 104. The wiper 102 spreads a fluid across a substrate held in position by the frame 104. The wiper 102 includes a handle 106 that enables a user to move the wiper 102 along the frame 104 while eventually spreading a fluid across the substrate held in place by the frame 104. The wiper 102 also includes a platform 108. The platform 108 includes an extension 110 extending from the handle 106 and a blade 112 extending downward from the extension 110 to engage the fluid. The platform 108 may be sized to contact at least a portion of a surface of the substrate or may be sized so as to not contact the surface of the substrate. The blade 112 may be straight, angled, or curved. The platform 108 may be composed of metal, ceramic, rubber, plastic, or a combination thereof. Alternatively, the extension 110 may be removed, and the blade 112 and the handle 106 may be longitudinally co-axial, thereby sharing the same central axis. Alternatively, the spreader 100 may allow for back and forth spreading, such that the wiper 102 is moved in a first direction and then is moved backwards in a second direction. For example, the blade 112 may include a double plough to spread the suspension in opposite directions.

The wiper 102 also includes a first wing 114 and a second wing 116. The first and second wings 114 and 116 include first and second flanges 118 and 120, respectively. The first and second flanges 118 and 120 are directed inward to mate with a first guide (not shown) and a second guide 130, respectively, located on opposite sides of the frame 104.

The frame 104 includes parallel first and second arms 122 and 124 that extend from a stanchion 126. The first and second arms 122 and 124 each include a shelf 128. The shelf 128 is a cut-out from an inner top portion of the respective side. The shelf 128 supports the substrate when the substrate is inserted into the frame 104. The shelf 128 inhibits translation of the substrate relative to the frame 104. The first and second arms 122 and 124 may be adjustable, thereby allowing for different dimensioned substrates to be used within the same frame 104.

The wiper 102 and the frame 104 may be interlockable via a tongue and groove connection to permit translation of the wiper 102 across, and with respect to, the frame 104. The first and second flanges 118 and 120 of the wiper 102 (i.e. the tongues) mate with a first guide (not shown) and a second guide 130 (i.e. the grooves), respectively. The first guide (not shown) and the second guide 130 include a linear portion 132 and a ramp 134. The linear portion 132 permits the wiper 102 to drag the fluid across the substrate at a consistent height. When the wiper 102 reaches and moves across the ramp 134, the blade 112 is lifted away from the substrate to avoid spreading off of the edges and end of the substrate while also allowing for ease of removal of the substrate from the frame 104. The ramp 134 may be angled or curved. The stanchion 126 may support at least a portion of the wiper 102 when the wiper 102 reaches the end of the ramp 134 in such a manner so as to prevent the blade 112 and/or the extension 110 from touching the stanchion 126 or any other portion of the frame 104. Alternatively, movement of the wiper 102 relative to the frame 104 may be constrained to the x-y plane, such as by a flexure mechanism, motorized actuator, linear or non-linear guides, a rotary mechanism, rack-and-pinion, or the like. Alternatively, the flanges may be located on the frame 104 whereas the guides may be located on the wiper 102.

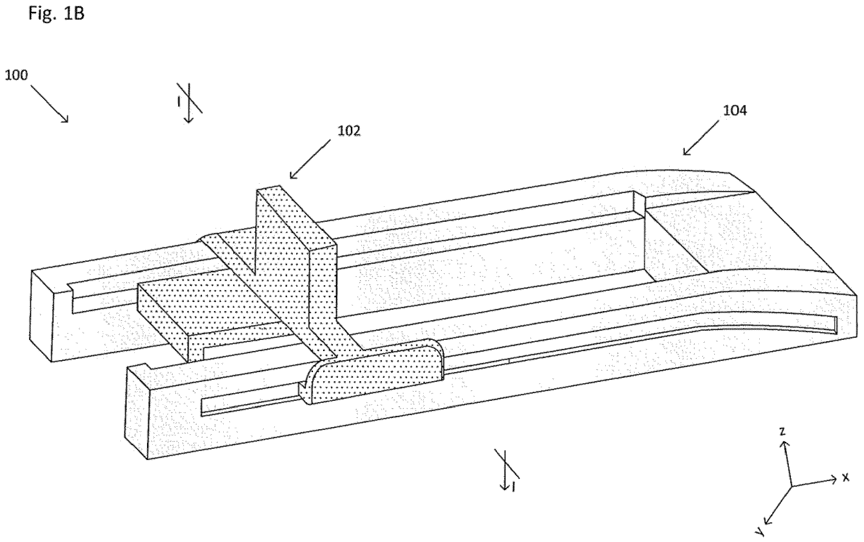

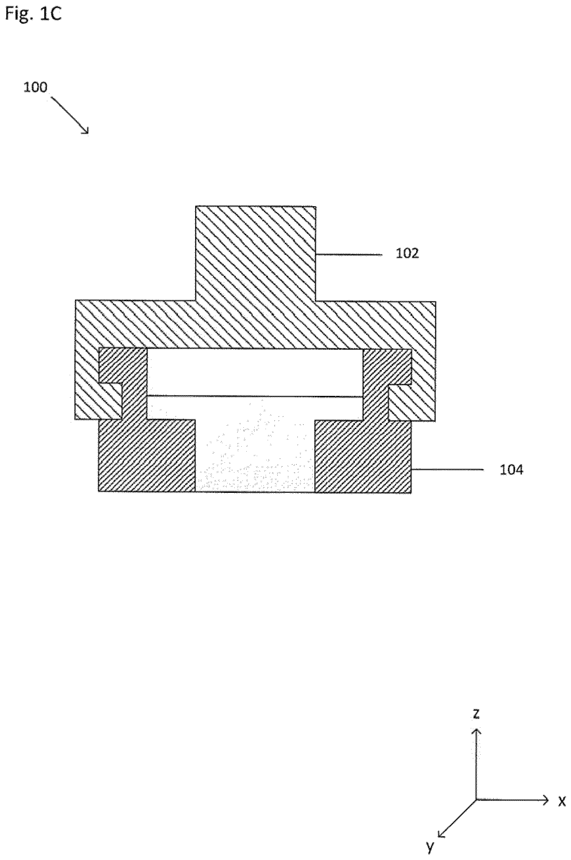

Figure 1B shows an isometric view of the spreader 100. Figure 1C shows a cross-section of the spreader 100 taken along the line I-I.

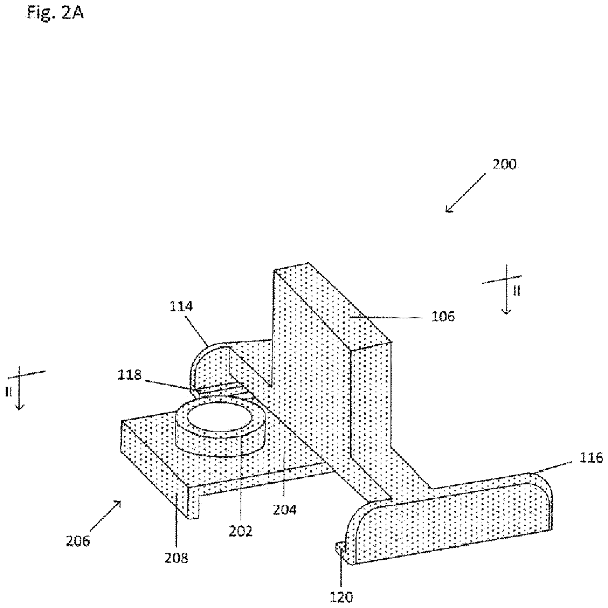

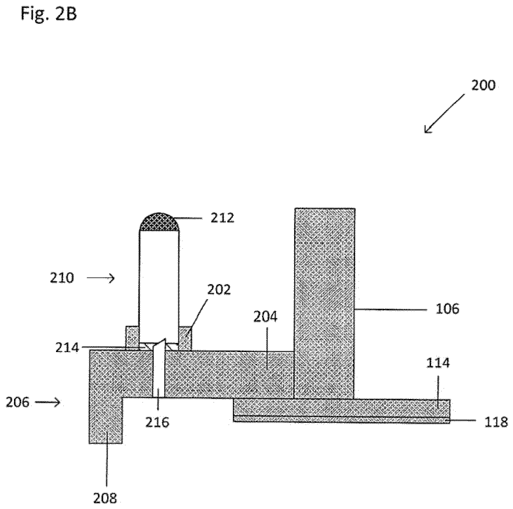

Figure 2A shows an example of a wiper 200. The wiper 200 is similar to the wiper 102, except that the wiper 200 includes a connector 202. The connector 202 may be located on a extension 204 of a platform 206. The platform 206 also includes a blade 208 similar to the blade 112. The connector 2C2 permits a vessel (not shown) to dispense a fluid onto the substrate without having to transfer the fluid or portion thereof to the substrate before being inserted into the frame. The connector 202 may be threaded or embedded with a Luer lock. The connector 202 may be any appropriate shape, including, but not limited to cylindrical, rectangular, conical, pyramidal, or the like. The connector 202 may be a hollow receptacle or may be a receptacle with a needle. For example, as seen in Figure 2B which is a cross-sectional view taken along the line II-II, a tube 210 with a flexible bottom 212 may be inverted such that a cap 214 is punctured by a needle 216. The flexible bottom is pushed, which draws a pre-determined volume of fluid through the needle. The fluid is then dispensed onto the substrate from the needle 216. Alternatively, the fluid may be dispensed via a syringe, a compressible cap, a pump via a fitting, or the like.

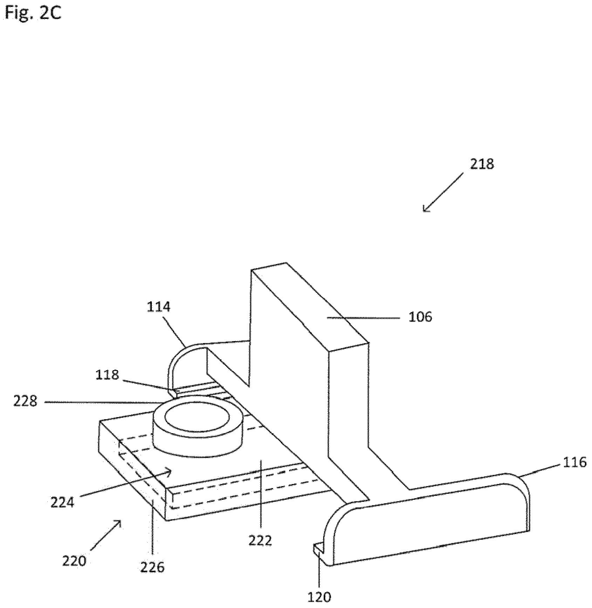

Figure 2C shows an example of a wiper 218. The wiper 218 is similar to the wiper 200, except that the wiper 218 includes a platform 220 where the extension 222 is enclosed on the sides to form a cavity 224 on an underside of the platform 220. The extension 222 also includes a blade 226 similar to the blade 112. The extension 222 may also include a connector 228 similar to the connector 202.

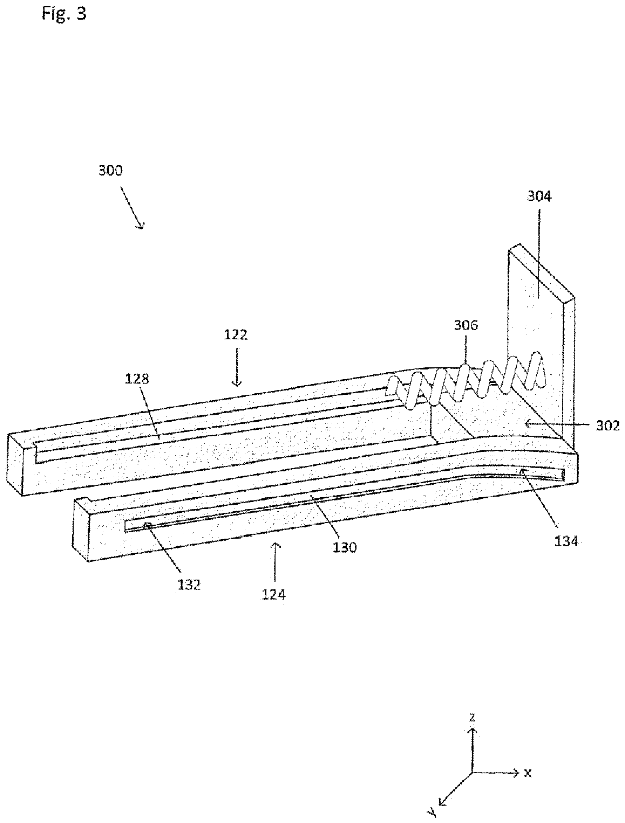

Figure 3 shows an example of a frame 300. The frame 300 is similar to the frame 104, except that the frame 300 includes a stanchion 302 with a back plate 304. The back plate 304 may include controllable drive mechanism 306 to move the wiper (not shown) along the frame 300 at a constant rate. The controllable drive mechanism 306 includes, but is not limited to, a spring, a damper (e.g. a rotary or linear damper) to move the wiper (not shown) at a constant rate. The controllable drive mechanism 306 may be used to move the wiper (not shown) along the frame at a predictable motion profile as the fluid droplet changes size across the substrate.

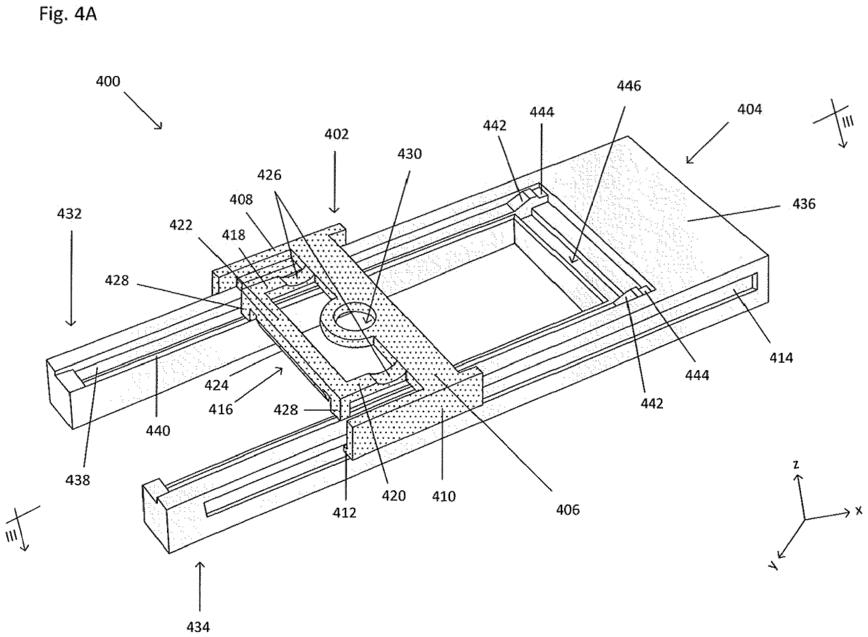

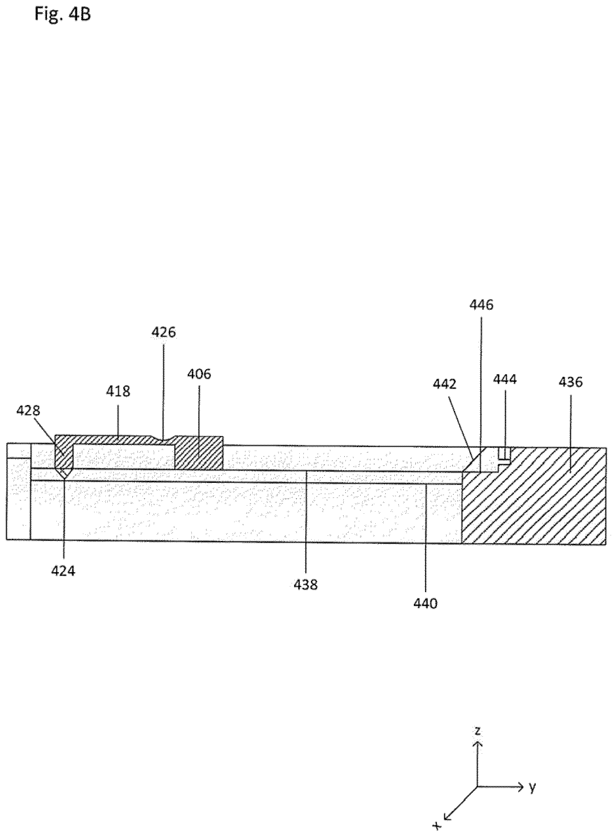

Figure 4A shows an isometric view of a spreader 400. Figure 4B shows a cross-sectional view of the spreader 400 taken along the line III-III. The spreader 400 includes a wiper 402 and a frame 404. The wiper 402 includes a main body 406 connected to a first wing 408 and a second wing 410. The first wing 408 includes a first flange (not shown) and the second wing 410 includes a second flange 412. The first flange (not shown) and the second flange 412 mate with a guide 414 of a respective side of the frame 404 (such as by a tongue-and-groove joint) to guide the x-, y-, and z-positions of the wiper 402. The first flange (not shown) and the second flange 412 extend towards the central axis of the main body 404 from the first and second wings 408 and 410, respectively.

The wiper 402 also includes a platform 416. The platform 416 includes first and second support members 418 and 420 extending from and connected to the main body 406. An extension 422 connects the first and second support members 418 and 420 at an end opposite the end at which the first and second support members 418 and 420 connect to the main body 406. The extension 422 includes a blade 424 to interact with a fluid on a substrate, thereby spreading the fluid across the substrate. The blade 424 may be hydrophobic or hydrophilic. The first and second support members 418 and 420 include flexures 426. The flexures 426 are cut-outs from the first and second support members 418 and 420 or flexible material to permit flexion of the blade 424 relative to the main body 406. The blade 424 may be sized to contact at least a portion of a surface of the substrate or may be sized so as to not contact the surface of the substrate. The blade 424 may be tapered or flat. The blade 424 may be composed of metal, ceramic, rubber, plastic, or a combination thereof. The wiper 402 also includes feet 428 at the junction of the extension 422 and the first and second support members 418 and 420, respectively, which extend downward and may engage the substrate to maintain a consistent blade height relative to the substrate. The flexures 426 may also be pre-loaded to keep the feet 428 in constant contact with the substrate. Alternatively, the feet 428 may engage a shelf of the frame 404.

The wiper 402 may also include a connector 430. The connector 430 may be located in the main body 406. The connector 430 permits a vessel (not shown) to dispense a fluid onto the substrate without having to transfer the fluid or portion thereof to the substrate before being inserted into the frame. The connector 430 may be a hole, a hollow receptacle, or may be a receptacle with a needle. For example, a tube or tubular main body with a flexible bottom may be inverted such that a cap is punctured by the needle. The flexible bottom is pushed, which draws a pre-determined volume of fluid through the needle. The fluid is then dispensed onto the substrate from the needle. Alternatively, the fluid may be dispensed via a syringe, a compressible cap, a pump via a fitting, or the like.

The connector 430 may be threaded or embedded with a Luer lock. The connector 430 may be any appropriate shape, including, but not limited to cylindrical, rectangular, conical, pyramidal, or the like. The vessel, upon connection with the connector 430, may be used as a handle. Alternatively, the wiper 402 may also include handle to move the wiper 402 relative to the frame 404.

The frame 404 includes a first arm 432 and a second arm 434, the first and second arms 432 and 434 being disconnected at one end and connected at another end via a stanchion 436. The first and second arms 432 and 434 each include a shelf 438. The shelf 438 is a cut-out from an inner top portion of the respective side. A lip 440 is then cut out from the respective shelf 438 within each side. The lip 440 supports the substrate when the substrate is inserted into the frame 404, whereas the shelf 438 supports the feet 428 of the platform 416. The lip 440 inhibits translation of the substrate relative to the frame 404. The first and second arms 432 and 434 may be adjustable, thereby permitting different dimensioned substrates to be used within the same frame 404.

The respective guides 414 are linear. Each shelf 438 also includes a riser 442 that lifts the platform 416 away from the substrate when the wiper 402 reaches the end of the shelf 438. The feet 428 of the platform 416 then rest in a detent 444 located behind each riser 442. A dock 446 supports at least a portion of the wiper 402, such as the blade 424, when the feet 428 reach the detents 444. Alternatively, the guides may be located on the wiper 402 whereas the flanges may be located on the frame 404. The wiper 402 may be moved relative to the frame 404 manually or mechanically by, including, but not limited to, a flexure mechanism, motorized actuator, linear or non-linear guides, a rotary mechanism, or the like.

The frame 404 may also include a back plate. The back plate may include a controllable drive mechanism to move the wiper 402 along the frame 404 at a constant rate. The controllable drive mechanism includes, but is not limited to, a spring, a damper (e.g. a rotary or linear damper) to move the wiper (not shown) at a constant rate. The controllable drive mechanism may be used to move the wiper (not shown) along the frame at a predictable motion profile as the fluid droplet changes size across the substrate.

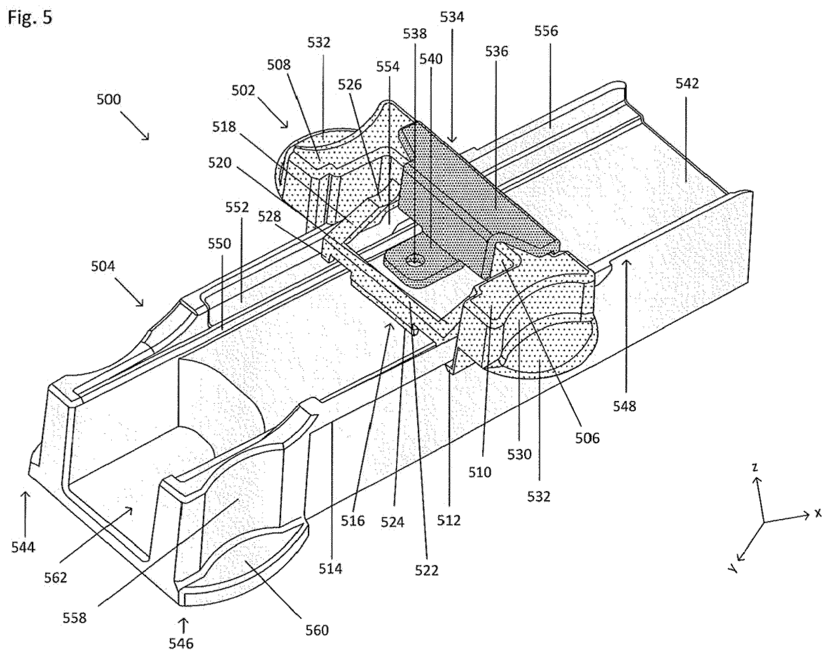

Figure 5 shows an isometric view of a spreader 500, The spreader 500 includes a wiper 502 and a frame 504, The wiper 502 includes a main body 506 connected to a first wing 508 and a second wing 510. Each wing 508 and 510 includes a flange 512. The flanges 512 mate with a guide 514 of a respective side of the frame 504 (such as by a tongue-and-groove joint) to guide the x-; y-, and z-positions of the wiper 502. The flanges 512 extend towards the central axis of the main body 504 from the first and second wings 508 and 510, respectively.

The wiper 502 also includes a platform 516, The platform 516 may include first and second support members 518 and 520 extending from and connected to the main body 504. An extension 522 connects the first and second support members 518 and 520 at an end opposite the end at which the first and second support members 518 and 520 connect to the main body 506. The extension 522 includes a blade 524 to interact with a fluid on a substrate, thereby spreading the fluid across the substrate. The blade 524 may be hydrophobic or hydrophilic. The first and second support members 518 and 520 include flexures 526. The flexures 526 are cut-outs from the first and second support members 518 and 520 or flexible material to permit flexion of the blade 524 relative to the main body 506. The blade 524 may be sized to contact at least a portion of a surface of the substrate or may be sized so as to not contact the surface of the substrate. The blade 524 may be metal, ceramic, rubber, plastic, or a combination thereof. The blade 524 may be tapered or flat. The wiper 502 also includes feet 528 at the junction of the extension 522 and the first and second support members 518 and 520, respectively, which extend downward and may engage the substrate to maintain a consistent blade height relative to the substrate. The flexures 526 may also be pre-loaded to keep the feet 528 in constant contact with the substrate. Alternatively, the feet 528 may engage a shelf of the frame 504. The first and second wings 508 and 510 may include a cut-out 530 to permit an operator or another device to grip the wiper 502 to be dragged across the frame 504. The first and second wings 508 and 510 may also include a guard 532 to prevent the operator or another device from disturbing other samples when at least two frames are placed side-by-side. The guard 532 may also permit for consistent grip height from wiper to wiper and when the wiper is moved from frame to frame.

The wiper 502 may also include a connector 534. The connector 534 may be a separate piece from the wiper 502 and may attach to the main body 506 of the wiper 502 via an attachment segment 536. The attachment segment 536 may be a clip so as to slip onto the main body 506. The connector 534 permits a vessel (not shown) to dispense a fluid onto the substrate without having to transfer the fluid or portion thereof to the substrate before being inserted into the frame. The connector 534 may include a bore 538 within a pedestal 540 extending from the attachment segment 536 towards the blade 542 of the wiper 502. The bore 538 may accept or mate with the vessel and may be threaded or may include a Luer lock. Alternatively, the bore 538 may include a needle to puncture the vessel. For example, a tube or tubular main body with a flexible bottom may be inverted such that a cap is punctured by the needle. The flexible bottom is pushed, which draws a pre-determined volume of fluid through the needle. The fluid is then dispensed onto the substrate from the needle. Alternatively, the fluid may be dispensed via a syringe, a compressible cap, a pump via a fitting, or the like.

The frame 504 includes a stanchion 542 which adjoins first and second sides 544 and 546. Each side of the frame 502 includes the guide 514, which may extend the full length of the respective side or may extend only a portion of the respective side. When the guide 514 only extends a portion of the respective side, the guide 514 may include a notch 548 to permit insertion of the wiper 502 onto the guide 514. The track 548 may be angled to maintain a relative distance of the blade 524 to the sample based on the changing volume of the not-yet-smeared portion of the sample (i.e. to maintain a consistent blade 524 to smear area ratio). Each flange 512 may be passed through the respective notch 548 and then moved to engage the respective guide 514. Each side also includes a shelf 550 to support the substrate when the substrate is inserted into the frame 504 and a mantle 552 to support and engage the feet 528 of the wiper 502. The mantle 552 may include a riser 554 to lift the blade 524 of the wiper 502 away from the substrate. The frame 504 may also include a lip 556 to inhibit translation of the substrate relative to the frame 504. Each side of the frame 504 may include a cut-out 558 to permit an operator or another device to grip the frame 504 while the wiper 502 is dragged across the frame 504. Each side of the frame 504 may also include a base 560 so that the operator or another device may stabilize the frame 504 by exerting a force on the frame 504. The frame 504 may also include a cavity 562 to permit the substrate to be inserted and removed from the frame 504 without touching the portion of the substrate that has or may receive the sample. The frame 504 may be level or angled (i.e. approximately 0.1°-5°).

The frame 504 may also include a back plate. The back plate may include controllable drive mechanism (not shown) to move the wiper 502 along the frame 504 at a constant rate. The controllable drive mechanism (not shown) includes, but is not limited to, a spring, a damper (e.g. a rotary or linear damper) to move the wiper (not shown) at a constant rate. The controllable drive mechanism (not shown) may be used to move the wiper (not shown) along the frame at a predictable motion profile as the fluid droplet changes size across the substrate.

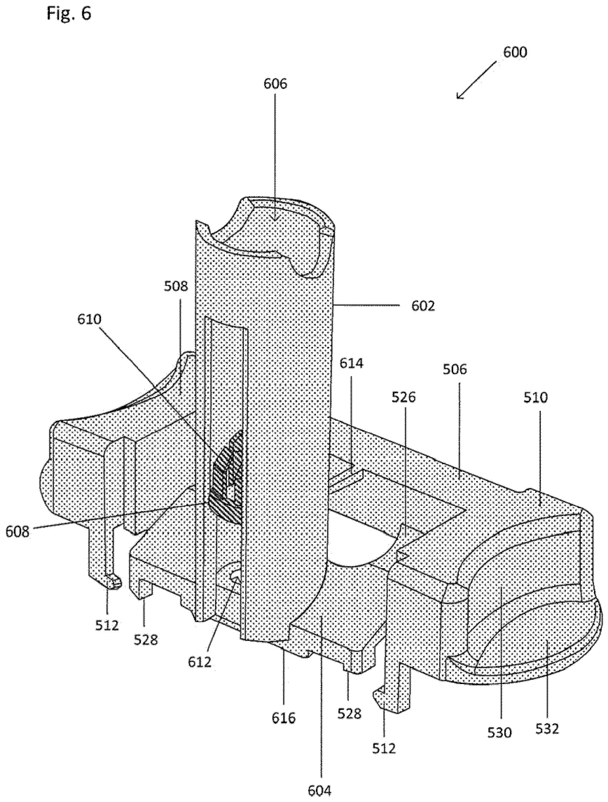

Figure 6 shows an isometric view of a wiper 600. The wiper 600 is similar to the wiper 502 except the wiper 600 includes a receptacle 602 extending from a brace 604. The receptacle 602 includes a bore 606 that spans the length of the receptacle and is configured to receive a vessel. The receptacle 602 may also include a collar 608, which may include fingers 610, to secure the vessel within the receptacle 602. The receptacle 602 holds the vessel containing a sample to be dispensed through an aperture 612. Alternatively, the receptacle 602 may include a needle to be inserted into the vessel to draw a portion of the sample out and onto the substrate. The needle extends vertically, thereby being parallel with the wall of the receptacle 602.

The wiper 600 may also include an abutment 614 that extends between the main body 506 and the receptacle 602 to support the receptacle 602. The brace 604 may be connected to the main body 506 via the flexures 526. The brace 604 includes a blade 616 similar to the blade 524.

The wiper may be composed of a variety of different materials including, but not limited to, a ceramic; metals; organic or inorganic materials; and plastic materials; and combinations thereof. The wiper may be disposable or re-useable. The frame may be composed of a variety of different materials including, but not limited to, a ceramic; metals; organic or inorganic materials; and plastic materials; and combinations thereof. The frame may be disposable or re-useable. The tracks may be on the outer side of the respective first and second sides or may be on the inner side of the respective first and second sides.

Alternatively, the spreader may include multiple frames lined up next to one another. A wiper may include multiple blades, such that the number of blades is equal to the number of frames. The blades may share a handle such that pulling on the handle causes all of the blades to move simultaneously. The blades may also share first and second arms so as to move off the substrates in unison.

Alternatively, the spreader may be housed in a cartridge to be inserted into a machine and then ejected from the machine after use. A lid of the cartridge may be removable to access the contents or a handle and connector may extend through the lid, so as to dispense the fluid onto the substrate and the move wiper to spread the fluid.

### Method

For the sake of convenience, the methods are described with reference to an example fluid of buffy coat. But the methods described below are not intended to be so limited in their scope of application. The methods, in practice, may be used with any kind of suspension, solution, or fluid. For example, a sample fluid may be urine, blood, Buffy coat, red blood cells, plasma, bone marrow, cystic fluid, ascites fluid, stool, semen, cerebrospinal fluid, nipple aspirate fluid, saliva, amniotic fluid, vaginal secretions, mucus membrane secretions, aqueous humor, vitreous humor, vomit, and any other physiological fluid or semi-solid. The method and apparatus may also be used with another appropriate solution or suspension. Furthermore, the example fluids may be mixed with a processing solution, such as a preservative, a cell adhesion solution, a fixative, or the like, from previous processing or for subsequent processing and analysis.

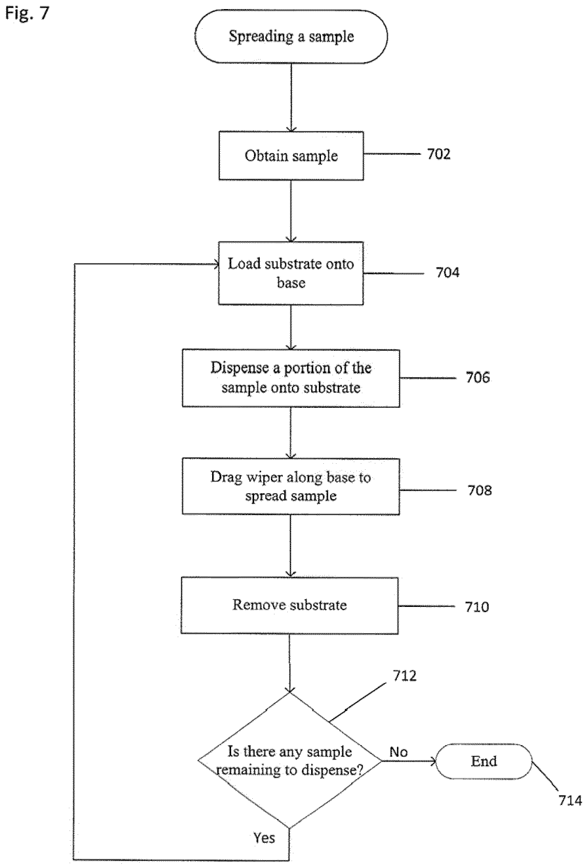

Figure 7 shows a flow diagram for spreading a sample with a spreader. In block 702, a sample of buffy coat may be obtained, such enrichment and/or isolation, and held within a vessel. In block 704, a substrate, such as a microscope slide, may be pre-loaded onto a shelf of a frame. In block 706, at least a portion of the buffy coat sample may be dispensed onto the substrate. The buffy coat may be dispensed in one drop or in more than one drop on the substrate. When one drop is dispensed onto the substrate, the drop may be approximately equidistant between the long sides of the substrate. When more than one drop is dispensed onto the substrate, the drops may be equally spaced apart between the long sides of the substrate.

In block 708, a wiper may be dragged across a frame to spread the sample on the substrate. The protrusion interacts with the buffy coat, thereby wicking the buffy coat outwards (i.e. width) and/or across (i.e. length) the microscope slide. When guides of the wiper slide up a ramp within a track of the frame, the protrusion is lifted away from the microscope slide. In block 710, the substrate may then be removed from the shelf either horizontally or vertically, so as to inhibit any run-off or movement of the buffy coat from the substrate.

In diamond 712, a determination may be made as to whether or not any sample remains in the vessel to be distributed. In oval 714, when no buffy coat remains, the process ends. However, when buffy coat remains, the process continues, starting at block 704 and a new substrate is placed onto the shelf, the wiper is reset, and the process is repeated as many times necessary until the vessel is empty.

The foregoing description, for purposes of explanation, used specific nomenclature to provide a thorough understanding of the disclosure. However, it will be apparent to one skilled in the art that the specific details are not required in order to practice the systems and methods described herein. The foregoing descriptions of specific embodiments are presented by way of examples for purposes of illustration and description. They are not intended to be exhaustive of or to limit this disclosure to the precise forms described. Many modifications and variations are possible in view of the above teachings. The embodiments are shown and described in order to best explain the principles of this disclosure and practical applications, to thereby enable others skilled in the art to best utilize this disclosure and various embodiments with various modifications as are suited to the particular use contemplated. It is intended that the scope of this disclosure be defined by the following claims:

## Claims

1. A system for spreading a fluid across a substrate, the
system comprising:
a wiper (402, 502, 600); and
a frame (404, 504) for supporting the wiper (402, 502, 600);
the frame (404, 504) having a first arm (432) or side (544) including a first guide (414, 514), a second arm (434) or side (546) including a second guide (414, 514), and a stanchion (436, 542) joining the first and second arms or sides, wherein each arm or side comprises a shelf (438, 550) for supporting a substrate;
the wiper (402, 502, 600) comprising:
a main body (406, 506) connected to a first wing (408, 508) and a second wing (410, 510), the first wing (408, 508) including a first flange (512) and the second wing (410, 510) including a second flange (412, 512), wherein the first flange (512) and the second flange (412, 512) mate with the first guide (414, 514) and the second guide (414, 514) respectively when the wiper is supported by the frame;
a platform (416, 516) comprising a first support member (418, 518) extending in a first direction from the main body (406, 506) and a second support member (420, 520) extending from the main body (406, 506) in the same direction as the first support member (418, 518);
an extension (422, 522) connecting the first and second support members (418, 420, 518, 520), the extension including a blade (424, 524) for extending toward the substrate to engage a fluid or a sample on the substrate; and
feet (428, 528) at the junction of the extension (422, 522) and the first and second support members (418, 420, 518, 520), respectively, which extend towards respective shelves (438, 550) when the wiper is supported by the frame;
**characterised in that**:
the first support member (418, 518) comprises at least one flexure (426, 526) and the second support member (420, 520) comprises at least one flexure (426, 526), wherein the flexures (426, 526) of the first and second support members are cut-outs from the first and second support members or flexible material; and
the flexures (426, 526) of the first and second support members (418, 420, 518, 520) are configured to permit flexion of the blade (424, 524) relative to the main body (406, 506).

2. The system of claim 1, the wiper further comprising a connector (534) to accept and hold a vessel at least partially filled with the fluid, the connector comprising:
an attachment segment (536) to attach to the main body (506) of the wiper (502);
a pedestal (540) extending away from the attachment segment and towards the blade (524) of the wiper (502); and
a bore (538) extending through the pedestal (540).

3. The system of claim 2, wherein the bore (538) of the connector (534) is threaded or includes a Luer lock.

4. The system of claim 2, wherein the bore (538) includes a needle to puncture the vessel, and preferably wherein the vessel comprises a tubular main body (210) with a flexible bottom (212) and a cap (214) to be punctured by the needle, such that when the flexible bottom is pushed, a pre-determined volume of fluid is drawn through the needle for dispensation onto the substrate from the needle.

5. The system of claim 1, wherein the blade (424, 524) of the wiper (402, 502, 600) is rectangular, semi-spherical, triangular, or tapered.

6. The system of claim 1, wherein the blade (424, 524) is rubber, plastic, ceramic, metal, or combinations thereof.

7. The system of claim 1, further comprising a connector (430) within the main body (406) to accept and hold a vessel at least partially filled with the fluid.

8. The system of claim 7, wherein the connector (430) is a hole, a receptacle, or a receptacle with a needle.

9. The system of claim 1, wherein each side (544, 546) of the frame (504) comprises a mantle (552) to support and engage the feet (528) of the wiper (502) when the wiper is supported by the frame, and preferably the frame (502) further comprises a lip (556) to inhibit translation of the substrate relative to the wiper (502).

10. The system of claim 9, wherein each mantle (552) includes a riser (554) to change the height of the feet (528) and lift the blade (524) away from the substrate.

11. The system of claim 1, wherein each shelf (438) includes:
a riser (442) to change the height of the feet (428) and lift the blade (424) away from the substrate; and
a detent (444) to lock in the feet (428) after traveling up the riser, and preferably wherein the platform includes a dock (446) to support the blade.

12. The system of claim 1, wherein the first and second guides (414, 514) extend a partial length of the first (432, 544) and second arms or sides (434, 546), respectively.

13. The system of claim 1, wherein the first and second guides (414, 514) are on the outer side of the first (432, 544) and second arms or sides (434, 546), respectively.

14. The system of claim 1, wherein the first arm (432) or side (544) has the first guide (414, 514) on an outer side and a said shelf (438, 550) on an inner side, wherein the second arm (434) or side (546) has the second guide (414, 514) on an outer side and a said shelf (438, 550) on an inner side, wherein the shelves of the first and second arms or sides extend towards one another, and wherein the first and second arms or sides are adjoined, extend parallel to one another, and extend in the same direction as one another.

15. The system of claim 14, wherein the frame (504) further comprises a first mantle (552) on the inner side of the first side (544) and a second mantle (552) on the inner side of the second side (546), wherein the first and second mantles are located above the shelves (550) on the first and second sides, and wherein a first said foot (528) engages the first mantle and a second said foot (528) engages the second mantle when the wiper (502) is supported by the frame.

## Patentansprüche

1. System zum Verteilen eines Fluids über ein Substrat, das System umfassend:
einen Wischer (402, 502, 600); und
einen Rahmen (404, 504) zum Tragen des Wischers (402, 502, 600);
wobei der Rahmen (404, 504) einen ersten Arm (432) oder eine erste Seite (544) mit einer ersten Führung (414, 514), einen zweiten Arm (434) oder eine zweite Seite (546) mit einer zweiten Führung (414, 514), und eine Stütze (436, 542), die den ersten und zweiten Arm bzw. die erste und zweite Seite verbindet, aufweist, wobei jeder Arm oder jede Seite ein Fach (438, 550) zum Tragen eines Substrats umfasst;
wobei der Wischer (402, 502, 600) Folgendes umfasst:
einen Hauptkörper (406, 506), der mit einem ersten Flügel (408, 508) und einem zweiten Flügel (410, 510) verbunden ist, wobei der erste Flügel (408, 508) einen ersten Flansch (512) beinhaltet und der zweite Flügel (410, 510) einen zweiten Flansch (412, 512) beinhaltet, wobei der erste Flansch (512) und der zweite Flansch (412, 512) mit der ersten Führung (414, 514) bzw. der zweiten Führung (414, 514) zusammenpassen, wenn der Wischer von dem Rahmen getragen wird;
eine Plattform (416, 516), umfassend ein erstes Stützelement (418, 518), das sich in einer ersten Richtung von dem Hauptkörper (406, 506) erstreckt, und ein zweites Stützelement (420, 520), das sich von dem Hauptkörper (406, 506) in der gleichen Richtung wie das erste Stützelement (418, 518) erstreckt;
eine Verlängerung (422, 522), die das erste und das zweite Stützelement (418, 420, 518, 520) verbindet, wobei die Verlängerung eine Lamelle (424, 524) beinhaltet, um sich in Richtung des Substrats zu erstrecken, um ein Fluid oder eine Probe auf dem Substrat einzugreifen; und
Füße (428, 528) an der Verbindung der Verlängerung (422, 522) und des ersten bzw. zweiten Stützelements (418, 420, 518, 520), die sich zu den jeweiligen Fächern (438, 550) erstrecken, wenn der Wischer von dem Rahmen getragen wird;
**dadurch gekennzeichnet, dass**:
das erste Stützelement (418, 518) mindestens eine Biegung (426, 526) umfasst und das zweite Stützelement (420, 520) mindestens eine Biegung (426, 526) umfasst, wobei die Biegungen (426, 526) des ersten und zweiten Stützelements Aussparungen aus dem ersten und zweiten Stützelement oder flexiblen Material sind; und
die Biegungen (426, 526) des ersten und zweiten Stützelements (418, 420, 518, 520) konfiguriert sind, um eine Biegung der Lamelle (424, 524) in Bezug auf den Hauptkörper (406, 506) zu ermöglichen.

2. System nach Anspruch 1, der Wischer ferner umfassend einen Verbinder (534) zum Aufnehmen und Halten eines Gefäßes, das zumindest teilweise mit dem Fluid gefüllt ist, wobei der Verbinder Folgendes umfasst:
ein Befestigungssegment (536) zum Befestigen an dem Hauptkörper (506) des Wischers (502);
einen Sockel (540), der sich weg von dem Befestigungssegment und in Richtung der Lamelle (524) des Wischers (502) erstreckt; und
eine Bohrung (538), die sich durch den Sockel (540) erstreckt.

3. System nach Anspruch 2, wobei die Bohrung (538) des Verbinders (534) eine Gewindebohrung ist oder einen Luer-Lock beinhaltet.

4. System nach Anspruch 2, wobei die Bohrung (538) eine Nadel zum Durchstechen des Gefäßes beinhaltet, und vorzugsweise wobei das Gefäß einen rohrförmigen Hauptkörper (210) mit einem flexiblen Boden (212) und einer Kappe (214) umfasst, die von der Nadel durchstochen werden soll, sodass beim Drücken des flexiblen Bodens ein vorbestimmtes Volumen an Fluid durch die Nadel gezogen wird, um von der Nadel auf das Substrat abgegeben zu werden.

5. System nach Anspruch 1, wobei die Lamelle (424, 524) des Wischers (402, 502, 600) rechteckig, halbkugelförmig, dreieckig oder kegelförmig ist.

6. System nach Anspruch 1, wobei die Lamelle (424, 524) aus Gummi, Kunststoff, Keramik, Metall oder Kombinationen davon ist.

7. System nach Anspruch 1, ferner umfassend einen Verbinder (430) innerhalb des Hauptkörpers (406) zum Aufnehmen und Halten eines Gefäßes, das zumindest teilweise mit dem Fluid gefüllt ist.

8. System nach Anspruch 7, wobei der Verbinder (430) ein Loch, eine Aufnahme oder eine Aufnahme mit einer Nadel ist.

9. System nach Anspruch 1, wobei jede Seite (544, 546) des Rahmens (504) eine Hülle (552) zum Tragen und Eingreifen der Füße (528) des Wischers (502) umfasst, wenn der Wischer durch den Rahmen getragen wird, und vorzugsweise der Rahmen (502) ferner eine Lippe (556) umfasst, um eine Translation des Substrats in Bezug auf den Wischer (502) zu verhindern.

10. System nach Anspruch 9, wobei jede Hülle (552) ein Steigrohr (554) beinhaltet, um die Höhe der Füße (528) zu ändern und die Lamelle (524) weg von dem Substrat anzuheben.

11. System nach Anspruch 1, wobei jedes Fach (438) Folgendes beinhaltet:
eine Steigrohr (442) zum Ändern der Höhe der Füße (428) und zum Anheben der Lamelle (424) weg von dem Substrat; und
eine Raste (444), um die Füße (428) zu verriegeln, nachdem sie an dem Steigrohr nach oben gewandert sind, und vorzugsweise, wobei die Plattform einen Steg (446) zum Halten der Lamelle beinhaltet.

12. System nach Anspruch 1, wobei sich die ersten und zweiten Führungen (414, 514) über eine Teillänge des ersten Arms/Seite (432, 544) bzw. zweiten Arms/Seite (434, 546) erstrecken.

13. System nach Anspruch 1, wobei die erste und zweite Führung (414, 514) an der Außenseite des ersten Arms/Seite (432, 544) bzw. des zweiten Arms/Seite (434, 546) sind.

14. System nach Anspruch 1, wobei der erste Arm (432) oder die erste Seite (544) die erste Führung (414, 514) auf einer Außenseite und ein Fach (438, 550) auf einer Innenseite aufweist, wobei der zweite Arm (434) oder die zweite Seite (546) die zweite Führung (414, 514) auf einer Außenseite und ein Fach (438, 550) auf einer Innenseite aufweist, wobei sich die Fächer des ersten und zweiten Arms oder der ersten und zweiten Seite angrenzend sind, sich parallel zueinander erstrecken und sich untereinander in die gleiche Richtung erstrecken.

15. System nach Anspruch 14, wobei der Rahmen (504) ferner eine erste Hülle (552) auf der Innenseite der ersten Seite (544) und eine zweite Hülle (552) auf der Innenseite der zweiten Seite (546) umfasst, wobei sich die erste und die zweite Hülle über den Fächern (550) auf der ersten und zweiten Seite befinden, und wobei ein erster Fuß (528) die erste Hülle eingreift und ein zweiter Fuß (528) die zweite Hülle eingreift, wenn der Wischer (502) von dem Rahmen getragen wird.

## Revendications

1. Système pour étaler un fluide sur un substrat, le système comprenant :
une racle (402, 502, 600) ; et
un cadre (404, 504) pour supporter la racle (402, 502, 600) ;
le cadre (404, 504) ayant un premier bras (432) ou un côté (544) comprenant un premier guide (414, 514), un deuxième bras (434) ou un côté (546) comprenant un deuxième guide (414, 514), et une colonne (436, 542) joignant les premier et second bras ou côtés, chaque bras ou côté comprenant une étagère (438, 550) pour supporter un substrat ;
la racle (402, 502, 600) comprenant :
un corps principal (406, 506) relié à une première aile (408, 508) et à une deuxième aile (410, 510), la première aile (408, 508) comprenant un premier rebord (512) et une deuxième aile (410, 510) comprenant un deuxième rebord (412, 512), le premier rebord (512) et le deuxième rebord (412, 512) s'accouplant avec le premier guide (414, 514) et le deuxième guide (414, 514) respectivement lorsque la racle est soutenue par le cadre ;
une plate-forme (416, 516) comprenant un premier élément de support (418, 518) s'étendant dans une première direction depuis le corps principal (406, 506) et un second élément de support (420, 520) s'étendant depuis le corps principal (406, 506) dans le même sens que le premier élément de support (418, 518) ;
une extension (422, 522) reliant les premier et second éléments de support (418, 420, 518, 520), l'extension comprenant une lame (424, 524) destinée à s'étendre vers le substrat pour venir en prise avec un fluide ou un échantillon sur le substrat ; et
des pieds (428, 528) à la jonction de l'extension (422, 522) et des premier et second éléments de support (418, 420, 518, 520), qui s'étendent respectivement vers les étagères respectives (438, 550) lorsque la racle est soutenue par le cadre ;
**caractérisé en ce que** :
le premier élément de support (418, 518) comprend au moins un élément de flexion (426, 526) et le second élément de support (420, 520) comprend au moins un élément de flexion (426, 526), dans lequel les éléments de flexion (426, 526) des premier et second éléments de support sont des découpes des premier et second éléments de support ou un matériau flexible ; et
les éléments de flexion (426, 526) des premier et second éléments de support (418, 420, 518, 520) sont configurées pour permettre la flexion de la lame (424, 524) par rapport au corps principal (406, 506).

2. Système selon la revendication 1, la racle comprenant en outre un connecteur (534) pour accepter et maintenir un récipient au moins partiellement rempli du fluide, le connecteur comprenant :
un segment de fixation (536) destiné à être fixé au corps principal (506) de la racle (502) ;
un socle (540) s'étendant à distance du segment de fixation et en direction de la lame (524) de la racle (502) ; et
un alésage (538) traversant le socle (540).

3. Système selon la revendication 2, dans lequel l'alésage (538) du connecteur (534) est fileté ou comprend un verrou Luer.

4. Système selon la revendication 2, dans lequel l'alésage (538) comprend une aiguille pour perforer le vaisseau, et de préférence dans lequel le vaisseau comprend un corps principal tubulaire (210) avec un fond flexible (212) et un bouchon (214) à perforé par l'aiguille, de telle sorte que lorsque le fond flexible est poussé, un volume prédéterminé de fluide est aspiré à travers l'aiguille pour distribution sur le substrat à partir de l'aiguille.

5. Système selon la revendication 1, dans lequel la lame (424, 524) de la racle (402, 502, 600) est rectangulaire, hémisphérique, triangulaire ou effilée.

6. Système selon la revendication 1, dans lequel la lame (424, 524) est en caoutchouc, en plastique, en céramique, en métal ou en une combinaison de ceux-ci.

7. Système selon la revendication 1, comprenant en outre un connecteur (430) à l'intérieur du corps principal (406) pour accepter et maintenir un récipient au moins partiellement rempli du fluide.

8. Système selon la revendication 7, dans lequel le connecteur (430) est un trou, un réceptacle ou un réceptacle avec une aiguille.

9. Système selon la revendication 1, dans lequel chaque côté (544, 546) du cadre (504) comprend un manteau (552) pour supporter et engager les pieds (528) de la racle (502) lorsque la racle est supportée par le cadre et, de préférence, le cadre (502) comprend en outre une lèvre (556) pour empêcher la translation du substrat par rapport à la racle (502).

10. Système selon la revendication 9, dans lequel chaque manteau (552) comprend une colonne montante (554) pour modifier la hauteur des pieds (528) et soulever la lame (524) pour l'éloigner du substrat.

11. Système selon la revendication 1, dans lequel chaque étagère (438) comprend :
une colonne montante (442) pour modifier la hauteur des pieds (428) et soulever la lame (424) pour l'éloigner du substrat ; et
une détente (444) pour verrouiller les pieds (428) après avoir remonté la colonne montante, et de préférence dans laquelle la plate-forme comprend un dock (446) pour supporter la lame.

12. Système selon la revendication 1, dans lequel les premier et second guides (414, 514) s'étendent sur une longueur partielle des premier (432, 544) et second bras ou côtés (434, 546), respectivement.

13. Système selon la revendication 1, dans lequel les premier et second guides (414, 514) sont sur le côté extérieur des premier (432, 544) et second bras ou côtés (434, 546), respectivement.

14. Système selon la revendication 1, dans lequel le premier bras (432) ou le côté (544) a le premier guide (414, 514) sur un côté extérieur et une étagère (438, 550) sur un côté intérieur, dans lequel un bras (434) ou un côté (546) a le deuxième guide (414, 514) sur un côté extérieur et une étagère (438, 550) sur un côté intérieur, les étagères des premier et deuxième bras ou côtés s'étendant les uns vers les autres, et dans lequel les premier et second bras ou côtés sont joints, s'étendent parallèlement les uns vers les autres et s'étendent dans la même direction les uns les autres.

15. Système selon la revendication 14, dans lequel le cadre (504) comprend en outre un premier manteau (552) du côté intérieur du premier côté (544) et un deuxième manteau (552) du côté intérieur du second côté (546), dans lequel les premier et deuxième manchons sont situés au-dessus des étagères (550) sur les premier et deuxième côtés, et dans lequel un dit premier pied (528) engage le premier manchon et un second pied (528) engage le deuxième manchon lorsque la racle (502) est soutenue par le cadre.
